(19) Europäisches Patentamt
European Patent Office
Office européen des brevets

(11) Veröffentlichungsnummer : **0 076 370**
**B1**

(12) # EUROPÄISCHE PATENTSCHRIFT

(45) Veröffentlichungstag der Patentschrift : **28.05.86**

(21) Anmeldenummer : **82107071.1**

(22) Anmeldetag : **05.08.82**

(51) Int. Cl.⁴ : **C 07 D249/08**, C 07 D233/60, A 01 N 43/64, A 01 N 43/50// C07C131/00

(54) **Substituierte Azolyl-phenoxy-Derivate, Verfahren zu ihrer Herstellung und ihre Verwendung als Fungizide.**

(30) Priorität : **17.08.81 DE 3132335**

(43) Veröffentlichungstag der Anmeldung : **13.04.83 Patentblatt 83/15**

(45) Bekanntmachung des Hinweises auf die Patenterteilung : **28.05.86 Patentblatt 86/22**

(84) Benannte Vertragsstaaten : **AT BE CH DE FR GB IT LI NL SE**

(56) Entgegenhaltungen :
**EP-A- 0 001 571**
**DE-A- 2 201 063**
**DE-A- 2 324 010**
**DE-A- 2 325 156**
**DE-A- 2 333 354**
**DE-A- 2 600 799**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Stetter, Jörg, Dr.**
**Gellertweg 4**
**D-5600 Wuppertal 1 (DE)**
Erfinder : **Büchel, Karl Heinz, Prof. Dr.**
**Dabringhausener Strasse 42**
**D-5093 Burscheid (DE)**
Erfinder : **Frohberger, Paul-Ernst, Dr.**
**Willi-Baumeister-Strasse 5**
**D-5090 Leverkusen (DE)**
Erfinder : **Reinecke, Paul, Dr.**
**Lessingstrasse 11**
**D-5090 Leverkusen 3 (DE)**
Erfinder : **Brandes, Wilhelm, Dr.**
**Eichendorffstrasse 3**
**D-5653 Leichlingen (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 076 370 B1

**Beschreibung**

Die vorliegende Erfindung betrifft neue substituierte Azolyl-phenoxy-Derivate, mehrere Verfahren zu ihrer Herstellung und ihre Verwendung als Fungizide.

Es ist bereits bekannt geworden, daß im Phenylteil substituierte 3,3-Dimethyl-1-(imidazol-1-yl)- bzw. -(1,2,4-triazol-1-yl)-1-phenoxy-butan-2-one und -ole allgemein gute fungizide Eigenschaften aufweisen (vergleiche DE-B-22 01 063, DE-B-23 24 010, DE-A-23 25 156 und DE-A-23 33 354). Die Wirkung dieser Verbindungen ist jedoch, insbesondere bei niedrigen Aufwandmengen und -konzentrationen, nicht immer in allen Indikationsbereichen ganz befriedigend. Dasselbe gilt auch für strukturell weiter entfernt liegende Phenylazophenyloxy-triazolyl-O,N-acetale und für acylierte Triazolyl-O,N-acetale, die in Patentanmeldungen beschrieben sind (vgl. EP-A-O 001 571 und DE-A-26 00 799).

Es wurden neue substituierte Azolyl-phenoxy-Derivate der allgemeinen Formel (I)

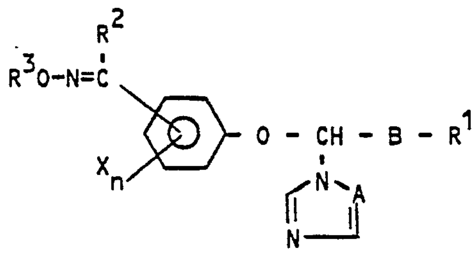

(I)

in welcher

A für ein Stickstoffatom oder die CH-Gruppe steht,

B für die Ketogruppe oder die CH (OH)-Gruppierung steht,

$R^1$ für Alkyl mit 1 bis 4 Kohlenstoffatomen und für Halogenalkyl mit 1 bis 4 Kohlenstoffatomen und 1 bis 3 Halogenatomen steht, sowie für gegebenenfalls durch Halogen, Alkyl mit 1 bis 4 Kohlenstoffatomen, Alkoxy und Alkylthio mit jeweils 1 bis 2 Kohlenstoffatomen und/oder Halogenalkyl mit 1 bis 2 Kohlenstoff- und 1 bis 5 gleichen oder verschiedenen Halogenatomen substituiertes Phenyl steht,

$R^2$ für Wasserstoff, für Alkyl mit 1 bis 4 Kohlenstoffatomen oder für gegebenenfalls durch die bei $R^1$ genannten Phenyl-Substituenten substituiertes Phenyl steht,

$R^3$ für Wasserstoff, für geradkettiges oder verzweigtes Alkyl mit 1 bis 4 Kohlenstoffatomen oder für Alkenyl und Alkinyl mit jeweils 2 bis 4 Kohlenstoffatomen steht,

X für Halogen, für Alkyl, Alkoxy und Alkylthio mit jeweils 1 bis 4 Kohlenstoffatomen oder für Halogenalkyl, Halogenalkoxy und Halogenalkylthio mit jeweils 1 bis 2 Kohlenstoffatomen und 1 bis 5 gleichen oder verschiedenen Halogenatomen steht, und

n für die Zahlen 0, 1 oder 2 steht,

sowie deren Säureadditions-Salze und Metallsalz-Komplexe gefunden.

Die Verbindungen der Formel (I) können in der syn- oder anti-Form vorliegen ; vorwiegend fallen sie als Gemische beider Formen an.

Diejenigen Verbindungen der Formel (I), in welchen B für die CH(OH)-Gruppierung steht, besitzen zwei asymmetrische Kohlenstoffatome ; sie können deshalb auch in den beiden geometrischen Isomeren (threo- und erythro- Form) vorliegen, die in unterschiedlichen Mengenverhältnissen anfallen können. In beiden Fällen liegen sie als optische Isomeren vor. Sämtliche Isomeren werden erfindungsgemäß beansprucht.

Weiterhin wurde gefunden, daß man die substituierten Azolyl-phenoxy-Derivate der Formel I erhält, wenn man

a) Halogenetherketone der Formel (II)

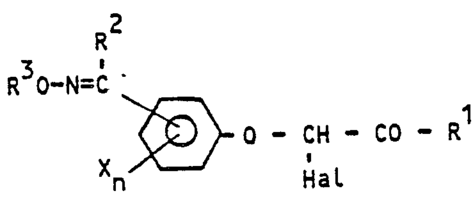

(II)

in welcher

$R^1$, $R^2$, $R^3$, X und n die oben angegebene Bedeutung haben und

Hal für Halogen steht,

mit 1,2,4-Triazol oder Imidazol, gegebenenfalls in Form von Alkalimetallsalzen, gegebenenfalls in Gegenwart eines Säurebindemittels und gegebenenfalls in Gegenwart eines Verdünnungsmittels umsetzt, oder

b) Azolylhalogenketone der Formel (III)

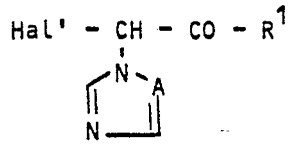

(III)

in welcher

A und $R^1$ die oben angegebene Bedeutung haben und

Hal' für Halogen steht,

mit Phenolen der Formel (IV)

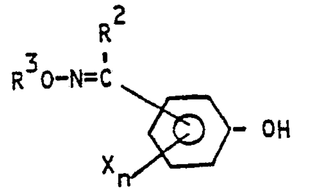

(IV)

in welcher $R^2$, $R^3$, X und n die oben angegebene Bedeutung haben, in Gegenwart eines Säurebindemittels und in Gegenwart eines Verdünnungsmittels, und gegebenenfalls

c) die nach den Verfahren (a) oder (b) erhaltenen Keto-Derivate der Formel (Ia)

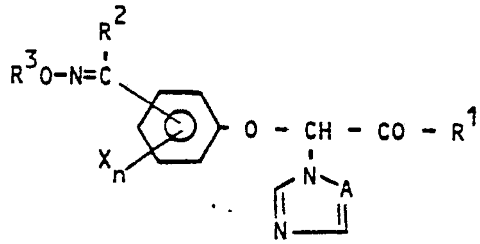

Ia

in welcher

$R^1$, $R^2$, $R^3$, A,

X und n die oben angegebene Bedeutung haben,

nach bekannten Methoden in üblicher Weise reduziert.

An die so erhaltenen Verbindungen der Formel (I) können gegebenenfalls anschließend eine Säure oder ein Metallsalz addiert werden.

Die neuen substituierten Azolyl-phenoxy-Derivate weisen starke fungizide Eigenschaften auf. Dabei zeigen überraschenderweise die erfindungsgemäßen Verbindungen eine höhere Wirkung als die aus dem Stand der Technik bekannten im Phenylteil substituierten 3,3-Dimethyl-1-(imidazol-1-yl)- bzw. (1,2,4-triazol-1-yl)-1-phenoxy-butan-2-one und -ole, welche chemisch und wirkungsmäßig naheliegendste Verbindungen sind. Die erfindungsgemäßen Stoffe stellen somit eine Bereicherung der Technik dar.

Die erfindungsgemäßen substituierten Azolyl-phenoxy-Derivate sind durch die Formel (I) allgemein definiert. In dieser Formel sind bevorzugt diejenigen Verbindungen der Formel (I), in denen

$R^1$ für tert.-Butyl, Chlor-tert.-butyl, Fluor-tert.-butyl, Dichlor-tert.-butyl, Difluor-tert.-butyl oder für gegebenenfalls einfach oder zweifach, gleich oder verschieden durch Fluor, Chlor und/oder Methyl substituiertes Phenyl steht;

$R^2$ für Wasserstoff, Methyl, Ethyl, Isopropyl, oder für gegebenenfalls einfach oder zweifach, gleich oder verschieden durch Fluor, Chlor, Methyl, Methoxy und/oder Trifluormethyl substituiertes Phenyl steht;

$R^3$ für Wasserstoff, Methyl, Ethyl, n-Propyl, n-Butyl, Allyl oder Propargyl steht;

X für Fluor, Chlor, Brom, Jod, Methyl, Methoxy, Methylthio, Trifluormethyl, Trifluormethoxy oder Trifluormethylthio steht, und

A, B, m und n für die in der Erfindungsdefinition angegebenen Bedeutungen stehen.

Im einzelnen seien außer den bei den Herstellungsbeispielen genannten Verbindungen die folgenden Verbindungen der allgemeinen Formel (I) genannt:

(Siehe Tabelle Seite 4 f.)

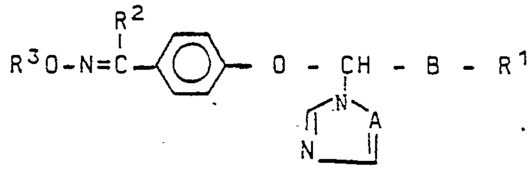 Ib

| $R^1$ | $R^2$ | $R^3$ | A | B |
|---|---|---|---|---|
| 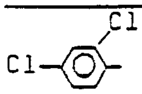 | H | $CH_3$ | CH | CO |
| 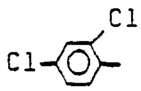 | H | $CH_3$ | N | CO |
| 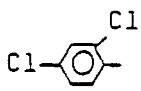 | H | $CH_3$ | CH | CHOH |
| 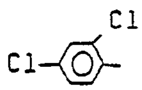 | H | $CH_3$ | N | CHOH |
| 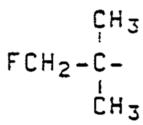 | H | $CH_3$ | CH | CO |
| 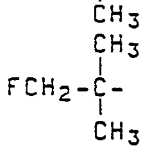 | H | $CH_3$ | N | CO |
| 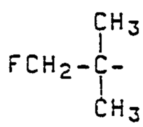 | H | $CH_3$ | CH | CHOH |
| 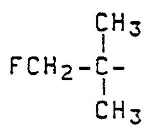 | H | $CH_3$ | N | CHOH |
| 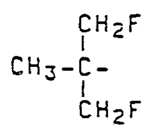 | H | $CH_3$ | CH | CO |
| 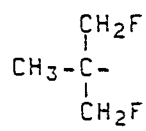 | H | $CH_3$ | N | CO |
| 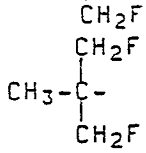 | H | $CH_3$ | CH | CHOH |
| 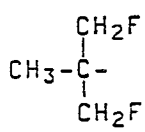 | H | $CH_3$ | N | CHOH |
| $(CH_3)_3C-$ | 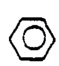 | $CH_3$ | CH | CO |
| $(CH_3)_3C-$ | 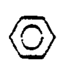 | $CH_3$ | N | CO |
| $(CH_3)_3C-$ | 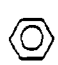 | $CH_3$ | CH | CHOH |
| $(CH_3)_3C-$ | 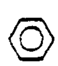 | $CH_3$ | N | CHOH |

Verwendet man beispielsweise 1-Brom-3,3-dimethyl-1-(4-methoxyiminomethylphenoxy)-butan-2-on und 1,2,4-Triazol als Ausgangsstoffe, so kann der Reaktionsablauf durch das folgende Formelschema wiedergegeben werden (Verfahren a) :

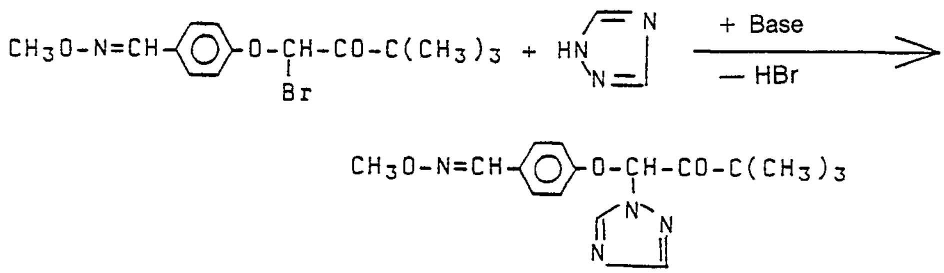


Verwendet man beispielsweise 1-Brom-3,3-dimethyl-1-(1,2,4-triazol-1-yl)-butan-2-on und 2-Hydroxy-benzaldehyd-O-methyl-oximether als Ausgangsstoffe, so kann der Reaktionsablauf durch das folgende Formelschema, wiedergegeben werden (Verfahren b) :

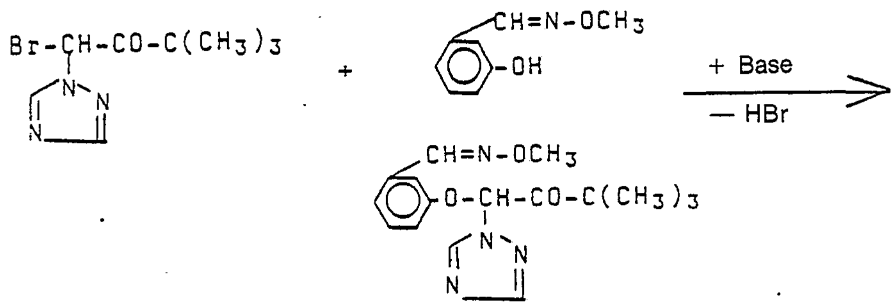


Verwendet man beispielsweise 3,3-Dimethyl-1-(4-methoxy-iminomethylphenoxy)-1-(1,2,4-triazol-1-yl)-butan-2-on und Natriumborhydrid als Ausgangsstoffe, so kann der Reaktionsablauf der Reduktion durch das folgende Formelschema wiedergegeben werden (Verfahren c) :

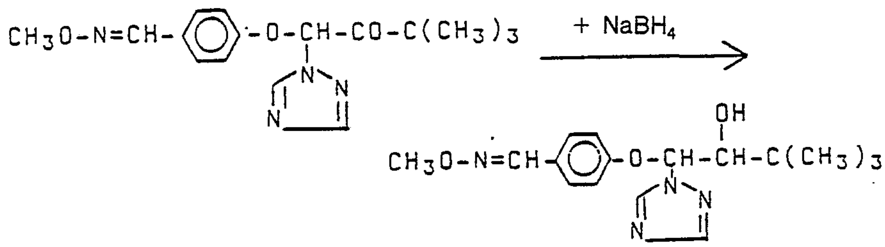


Die bei der Druchführung des erfindungsgemäßen Verfahrens (a) als Ausgangsstoffe zu verwendenden Halogenetherketone sind durch die Formel (II) allgemein definiert. In dieser Formel stehen $R^1$, $R^2$, $R^3$, X und der Index n vorzugsweise für diejenigen Bedeutungen, die bereits im Zusammenhang mit der Beschreibung der erfindungsgemäßen Stoffe der Formel (I) vorzugsweise für diese Reste genannt wurden. Hal steht vorzugsweise für Fluor, Chlor oder Brom.

Die Halogenetherketone der Formel (II) sind noch nicht bekannt. Sie können jedoch nach bekannten Verfahren erhalten werden, indem man Phenole der Formel (IV)

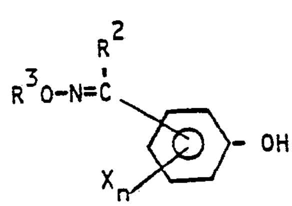

(IV)

in welcher $R^2$, $R^3$, X und n die oben angegebene Bedeutung haben mit Halogenketonen der Formeln (Va) bzw. (Vb)

$$Hal'—CH_2—CO—R^1 \qquad (Va)$$

bzw.

$$Hal'—CHF—CO—R^1 \qquad (Vb)$$

in welchen
Hal' für Chlor und Brom steht und
$R^1$ die oben angegebene Bedeutung hat,
in Gegenwart eines inerten organischen Lösungsmittels, wie z. B. Aceton, und in Gegenwart eines Säurebinders, wie z. B. Kaliumcarbonat, umsetzt. Bei Verwendung eines Halogenketons der Formel (Va) wird das verbliebene aktive Wasserstoffatom anschließend in üblicher Weise gegen Chlor oder Brom ausgetauscht.

Die Phenole der Formel (IV) sind bekannt, bzw. sie können in allgemein üblicher Art und Weise erhalten werden (vergleiche auch die Herstellungsbeispiele).

Die Halogenketone der Formeln (Va) und (Vb) sind bekannt (vergleiche z. B. DE-A-22 01 063, DE-A-23 25 156 und DE-A-29 37 595) bzw. können sie in allgemein bekannter Art und Weise erhalten werden. Die Halogenketone der Formel (Va) werden erhalten, indem man entsprechende Ketone mit Chlor oder Brom in Gegenwart eines inerten organischen Lösungsmittels, wie z. B. Ether oder chlorierte Kohlenwasserstoffe, bei Raumtemperatur verstzt, oder mit üblichen Chlorierungsmitteln, wie beispielsweise Sulfurylchlorid, bei 20 bis 60° C umsetzt. Die Halogenketone der Formel (Vb) werden erhalten, indem man in Halogenketonen der Formel (Va) das Brom bzw. Chlor in üblicher Weise gegen Fluor austauscht und in den entstehenden entsprechenden Fluorketonen eines der beiden aktiven. Wasserstoffatome in der o. g. Weise gegen Brom oder Chlor austauscht.

Die bei der Durchführung des erfindungsgemäßen Verfahrens (b) als Ausgangsstoffe zu verwendenden Azolylhalogenketone sind durch die Formel (III) allgemein definiert. In dieser Formel stehen $R^1$ und A vorzugsweise für diejenigen Bedeutungen, die bereits im Zusammenhang mit der Beschreibung der erfindungsgemäßen Stoffe der Formel (I) vorzugsweise für diese Reste genannt wurden. Hal' steht vorzugsweise für Fluor, Chlor oder Brom.

Die Azolylhalogenketone der Formel (III) sind bekannt (vergleiche DE-A-27 56 269 und DE-A-29 37 595), bzw. können sie nach den dort angegebenen Verfahren erhalten werden, indem man z. B. Azolylketone der Formel (VI)

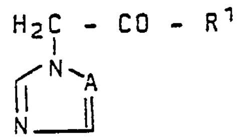
(VI)

in welcher A und $R^1$ die oben angegebene Bedeutung haben, mit Brom oder Chlor in Gegenwart eines sauren Lösungsmittels und gegebenenfalls in Gegenwart eines Halogenwasserstoffakzeptors umsetzt und gegebenenfalls in den entsprechenden Azolyl-brom(chlor)-ketonen das Brom(Chlor) in üblicher Weise gegen Fluor austauscht. Die so erhaltenen Azolylhalogenketone können ohne Isolierung direkt weiter umgesetzt werden.

Die Azolylketone der Formel (VI) sind bekannt (vergleiche DE-A-26 38 470, DE-A-24 31 407 und DE-A-28 20 361), bzw. können sie nach den dort angegebenen Verfahren erhalten werden, indem man Halogenketone der Formel

$$(Cl)Br—CH_2—CO—R^1 \qquad (VII)$$

in welcher $R^1$ die oben angegebene Bedeutung hat, mit 1,2,4-Triazol oder Imidazol in Gegenwart eines Verdünnungsmittels, wie beispielsweise Aceton, und in Gegenwart eines Säurebindemittels, wie beispielsweise Kaliumcarbonat, bei Temperaturen zwischen 20 und 150 °C umsetzt.

Für die erfindungsgemäße Umsetzung gemäß Verfahren (a) kommen als Verdünnungsmittel inerte organische Lösungsmittel infrage. Hierzu gehören vorzugsweise Ketone, wie Diethylketon und insbesondere Aceton und Methylethylketon ; Nitrile, wie Propionitril, insbesondere Acetonitril ; Alkohole, wie Ethanol oder Isopropanol ; Ether, wie Tetrahydrofuran oder Dioxan ; Benzol ; Toluol ; Formamide, wie insbesondere Dimethylformamid ; und halogenierte Kohlenwasserstoffe.

Die erfindungsgemäße Umsetzung gemäß Verfahren (a) wird gegebenenfalls in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan.

Vorzugsweise verwendet man einen entsprechenden Ueberschuß an Triazol bzw. Imidazol.

Die Reaktionstemperaturen können beim Verfahren (a) in einem größeren Bereich variiert werden. Im

allgemeinen arbeitet man zwischen 20 bis 150 °C, vorzugsweise bei 20 bis 120 °C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (a) setzt man auf 1 Mol der Verbindungen der Formel (II) vorzugsweise 2 Mol Triazol bzw. Imidazol und 1 bis 2 Mol Säurebinder ein. Zur Isolierung der Verbindungen der Formel (I) wird das Lösungsmittel abdestilliert, der Rückstand mit einem organischen Solvens aufgenommen und mit Wasser gewaschen. Die organische Phase wird über Natriumsulfat getrocknet und in Vakuum vom Lösungsmittel befreit. Der Rückstand wird durch Destillation bzw. Umkristallisation oder Salzbildung und Umkristallisation gereinigt.

In einer bevorzugten Ausführungsform des erfindungsgemäßen Verfahrens (a) können Verbindungen der Formel (II), in denen Hal für Fluor steht, auch direkt in der Schmelze bei Temperaturen zwischen 100 und 200 °C mit 1,2,4-Triazol oder Imidazol umgesetzt werden.

In einer weiteren bevorzugten Ausführungsform des erfindungsgemäßen Verfahrens (a) können Verbindungen der Formel (I), in denen B für die CH(OH)-Gruppierung steht, auch erhalten werden, indem man zunächst Halogenetherketone der Formel (II), in denen Hal für Fluor steht, erfindungsgemäß nach Verfahren (c) reduziert und anschließend erfindungsgemäß mit 1,2,4-Triazol oder Imidazol umsetzt.

Für die erfindungsgemäße Umsetzung gemäß Verfahren (b) kommen als Verdünnungsmittel vorzugsweise inerte organische Lösungsmittel infrage. Hierzu gehören vorzugsweise Ehter, wie Diethylether, Alkohole, wie Methanol ; Ketone, wie Aceton ; aromatische Kohlenwasserstoffe, wie Benzol ; sowie auch Dimethyl sulfoxid und Dimethylformamid.

Die erfindungsgemäße Umsetzung gemäß Verfahren (b) wird in Gegenwart eines Säurebinders vorgenommen. Man kann alle üblicherweise verwendbaren anorganischen oder organischen Säurebinder zugeben, wie Alkalimetallcarbonate, beispielsweise Kaliumcarbonat oder Natriumcarbonat, Alkalimetallhydroxide, beispielsweise Natriumhydroxid, Alkalimetallalkoholate, oder wie niedere tertiäre Alkylamine, beispielsweise Triethylamin.

Die Reaktionstemperaturen können bei der Durchführung des erfindungsgemäßen Verfahrens (b) in einem größeren Bereich variiert werden. Im allgemeinen arbeitet man zwischen 0 und 140 °C, vorzugsweise zwischen 50 und 100 °C.

Bei der Durchführung des erfindungsgemäßen Verfahrens (b) setzt man auf 1 Mol der Verbindung der Formel (III) vorzugsweise 1 bis 4 Mol Phenol der Formel (IV) ein. Die Isolierung der Verbindungen der Formel (I) erfolgt in üblicher Weise. Die Verbindungen der Formel (III) können vorzugsweise in Form ihrer Hydrohalogenide eingesetzt werden.

Die erfindungsgemäße Reduktion gemäß Verfahren (c) erfolgt in üblicher Weise, wie z. B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel infrage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30 °C, vorzugsweise bei 0 bis 20 °C durchgeführt. Hierzu setzt man auf 1 Mol des Ketons der Formel (Ia) etwa 1 Mol eines komplexen Hydrids, wie Natriumborhydrid oder Lithiumalanat, ein. Zur Isolierung der reduzierten Verbindungen der Formel (I) wird der Rückstand in verdünnter Salzsäure aufgenommen, anschließend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, infrage. Die Reaktionstemperaturen können wiederum in einem größeren Bereich variiert werden, im allgemeinen arbeitet man zwischen 20 und 120 °C, vorzugsweise zwischen 50 und 100 °C.

Zur Durchführung der Reaktion setzt man auf 1 Mol des Ketons der Formel (Ia) etwa 0,3 bis 2 Mol Aluminiumisopropylat ein. Zur Isolierung der reduzierten Verbindungen der Formel (I) wird das überschüssige Lösungsmittel im Vakuum entfernt und die entstandenen Aluminium-Verbindungen mit verdünnter Schwefelsäure oder Natronlauge zersetzt. Die weitere Aufarbeitung erfolgt in üblicher Weise.

Zur Herstellung von physiologisch verträglichen Säure-additionssalzen der Verbindungen der Formel (I) kommen vorzugsweise folgende Säuren infrage : Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionnelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure.

Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien. Als Anionen der

Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zur Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Getreidekrankheiten, wie gegen echten Gerstenmehltau (Erysiphe graminis) und Streifenkrankheiten der Gerste (Drechslera graminea und Pyrenophora teres) eingesetzt werden, sowie auch zur Bekämpfung von Rostkrankheiten, wie z. B. gegen den Erreger des Bohnenrostes (Uromyces appendiculatus), und von Schorfkrankheiten, wie gegen den Erreger des Apfelschorfs (Venturia inaequalis). Darüber hinaus können die erfindungsgemäßen Wirkstoffe auch mit gutem Erfolg gegen andere Pflanzenkrankheiten eingesetzt werden, die z. B. durch die Erreger der Pilzgattungen Puccinia, Leptosphaeria, Cochliobolus und Pyrenophora verursacht werden. Die erfindungsgemäßen Wirkstoffe zeigen auch ein gutes breites *in-vitro*-Spektrum.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsions-konzentrate, Saatgutpuder, Wirkstoff-imprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder Festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfs lösungsmittel verwendet werden. Als flüssige Lösungsmittel kommen im wesentlichen infrage : Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser ; mit verflüssigten gasförmigen Streckmitteln oder Trägerstoffen sind solche Flüssig-keiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid ; als feste Trägerstoffe kommen in Frage : z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln ; als Emulgier- und/oder schaumerzeugende Mittel kommen in Frage ; z. B. nichtionogene und anionische Emulgatoren, wie Polyoxyethylen-Fettsäureester, Polyoxyethylen-Fettalkohol-ether, z. B. Alkylarylpolyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxy-methycellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo-, Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischeno, 1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Fomulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einen größeren Bereich variiert werden. Sie liegen im allgemeinen zwischen 1 und 0,000 1 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,000 01 bis 0,1 Gew.-%, vorzugsweise von 0,000 1 bis 0,02 Gew.-%, am Wirkungsort erforderlich.

## Herstellungsbeispiele

### Beispiel 1

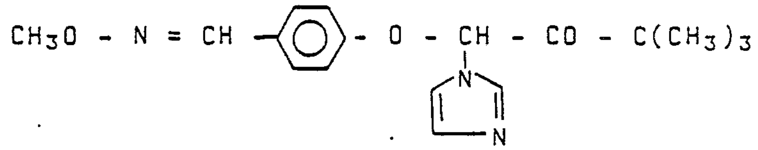

(Verfahren a)

Zu einer bei 150 °C gehaltenen Schmelze von 68 g (1 Mol) Imidazol werden unter Rühren 220 g (0,82 Mol) 3,3-Dimethyl-1-fluor-1-(4-methoximinomethyl-phenoxy)-butan-2-on getropft. Es wird 3 Stunden bei 150 °C nachgerührt, das Reaktionsgemisch anschließend auf Wasser gegeben und die wässrige Phase mehrmals mit Methylenchlorid ausgeschüttelt. Nach Trocknen über Natriumsulfat wird das Lösungsmittel im Vakuum entfernt. Es verbleibt ein dunkles Oel, das nach einiger Zeit völlig durchkristallisiert. Man erhält 210 g (81 % der Theorie) 3,3-Dimethyl-1-(imidazol-1-yl)-1-(4-methoximinomethyl-phenoxy)-butan-2-on vom Schmelzpunkt 73-80 °C.

Herstellung des Ausgangsproduktes

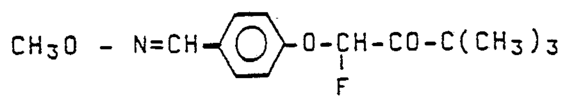

Zu einer Mischung aus 151 g (1 Mol) 4-Hydroxy-benzaldehyd-O-methyl-oximether und 154 g (1,1 Mol) fein gepulvertem Kaliumcarbonat in Aceton werden unter Rühren 217 g (1,1 Mol) 1-Brom-1-fluor-3,3-dimethyl-butan-2-on getropft. Nach Beendigung der leicht exothermen Reaktion wird noch 3 Stunden nachgeführt. Nach Abfiltrieren des anorganischen Niederschlages wird die Lösung eingeengt und der Rückstand im Vakuum destilliert. Man erhält 229 g (78 % der Theorie) 3,3-Dimethyl-1-fluor-1-(4-methoximinomethyl-phenoxy)-butan-2-on vom Siedepunkt 155 °C/0,1 Torr.

### Beispiel 2

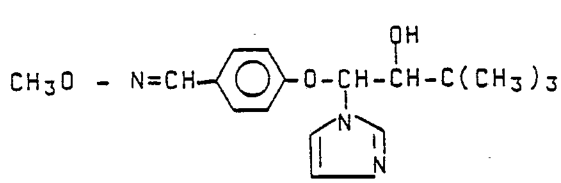

(Verfahren c)

5,65 g (0,018 Mol) 3,3-Dimethyl-1-(imidazol-1-yl)-1-(4-methoximinomethyl-phenoxy)-butan-2-on (Beispiel 1) werden in Methanol gelöst und bei Raumtemperatur portionsweise mit 2,3 g (0,06 Mol) Natriumborhydrid versetzt. Es wird 6 Stunden bei Raumtemperatur gerührt, das Reaktionsgemisch anschließend auf Wasser gegossen und mit Methylenchlorid extrahiert. Die Methylenchlorid-Phase wird über Natriumsulfat getrocknet und anschließend eingeengt. Man erhält 4,1 g (72 % der Theorie) 3,3-Dimethyl-1-(imidazol-1-yl)-1-(4-methoximinomethyl-phenoxy)-butan-2-ol vom Schmelzpunkt 37-45 °C.

Beispiel 3

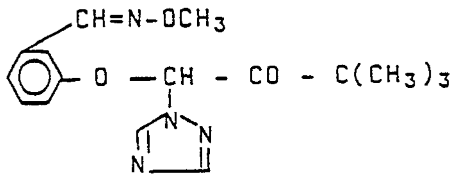

(Verfahren b)

10 g (0,06 Mol) 3,3-Dimethyl-1-(1,2,4-triazol-1-yl)-butan-2-on und 4,9 g (0,06 Mol) Natriumacetat werden in 100 ml Eisessig vorgelegt und unter Rühren bei 30-35 °C mit 9,6 g (0,06 Mol) Brom tropfenweise versetzt. Es wird 4 Stunden bis zum völligen Verschwinden der Bromfarbe nachgerührt; anschließend wird das Reaktionsgemisch auf Wasser gegossen und mit Chloroform ausgeschüttelt. Die Chloroform-Phase wird mit Natriumhydrogencarbonat neutralisiert und im Vakuum eingeengt. Das rohe 1-Brom-(1,2,4-triazol-1-yl)-3,3-dimethyl-butan-2-on wird bei Raumtemperatur zu einer gerührten Mischung aus 5,5 g (0,036 Mol) 3-Hydroxy-benzaldehyd-O-methyl-oximether und 8,35 g (0,06 Mol) Kaliumcarbonat getropft. Nach Abklingen der leicht exothermen Reaktion wird 3 Stunden bei Raumtemperatur gerührt, anschließend der Niederschlag abgesaugt und die Lösung im Vakuum vom Lösungsmittel befreit. Der Rückstand kristallisiert nach Verreiben mit Petrolether. Man erhält 11,25 g (98 % der Theorie) 3,3-Dimethyl-1-(3-methoximino-methyl-phenoxy)-1-(1,2,4-triazol-1-yl)-butan-2-on vom Schmelzpunkt 89-95 °C.

Herstellung des Ausgangsproduktes

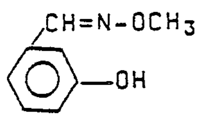

Eine Mischung aus 24,4 g (0,2 Mol) 3-Hydroxy-benzaldehyd, 18,4 g (0,22 Mol) O-Methylhydroxylaminhydrochlorid und 22,2 g (0,22 Mol) Triethylamin wird in 200 ml Ethanol 4 Stunden unter Rückfluß erhitzt. Nach Abdampfen des Lösungsmittels wird der Rückstand zwischen Wasser und Methylenchlorid verteilt, die organische Phase über Natriumsulfat getrocknet und eingeengt. Man erhält 23,1 g (77 % der Theorie) 3-Hydroxy-benzaldehyd-O-methyl-oximether vom Schmelzpunkt 50-56 °C.

In analoger Weise und entsprechend den erfindungsgemäßen Verfahren werden die in der nachfolgenden Tabelle aufgeführten Verbindungen der allgemeinen Formel (I)

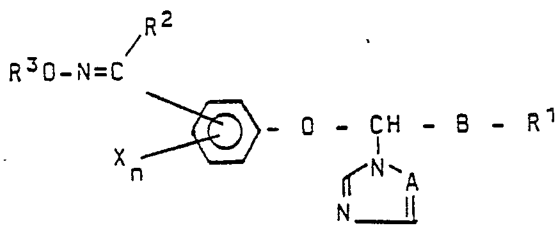 (I)

erhalten:

| Bsp. Nr. | $R^3O-N=C(R^2)-$ | $X_n$ | A | B | $R^1$ | Schmelzpunkt (°C) |
|---|---|---|---|---|---|---|
| 4 | 4-CH=N-OCH$_3$ | - | N | CO | C(CH$_3$)$_3$ | zähfl. Oel |
| 5 | 2-CH=N-OCH$_3$ | 4,6-Cl$_2$ | N | CO | C(CH$_3$)$_3$ | 93-98 |
| 6 | 3-CH=N-OCH$_3$ | 5-OCH$_3$ | N | CO | C(CH$_3$)$_3$ | 91-96 |
| 7 | 4-C(CH$_3$)=N-OCH$_3$ | - | N | CO | C(CH$_3$)$_3$ | 83-90 |
| 8 | 2-CH=N-OCH$_3$ | 4-Br | N | CO | C(CH$_3$)$_3$ | 131-37 |
| 9 | 2-CH=N-OCH$_3$ | - | N | CO | C(CH$_3$)$_3$ | 55-67 |
| 10 | 4-CH=N-OCH$_3$ | 2-OCH$_3$ | N | CO | C(CH$_3$)$_3$ | 93-110 |

(Fortsetzung)

| Bsp. Nr. | $R^3O-N=\overset{R^2}{C}-$ | $X_n$ | A | B | $R^1$ | Schmelzpunkt(°C) |
|---|---|---|---|---|---|---|
| 11 | $4-CH=N-OCH_3$ | - | N | CH(OH) | $C(CH_3)_3$ | zähfl.Oel |
| 12 | $2-CH=N-OCH_3$ | $4,6-Cl_2$ | N | CH(OH) | $C(CH_3)_3$ | 105-24 |
| 13 | $3-CH=N-OCH_3$ | $5-OCH_3$ | N | CH(OH) | $C(CH_3)_3$ | zähfl.Oel |
| 14 | $2-CH=N-OCH_3$ | 4-Br | N | CH(OH) | $C(CH_3)_3$ | 52-70 |
| 15 | $4-\underset{CH_3}{C}=N-OCH_3$ | - | N | CH(OH) | $C(CH_3)_3$ | 121-36 |
| 16 | $2-CH=N-OCH_3$ | - | N | CH(OH) | $C(CH_3)_3$ | 105-41 |
| 17 | $3-CH=N-OCH_3$ | - | N | CH(OH) | $C(CH_3)_3$ | zähfl.Oel |
| 18 | $4-CH=N-OCH_3$ | $2-OCH_3$ | N | CH(OH) | $C(CH_3)_3$ | 111-40 |
| 19 | $3-CH=N-OCH_3$ | - | CH | CO | $C(CH_3)_3$ | zähfl.Oel |
| 20 | $3-CH=N-OCH_3$ | - | CH | CH(OH) | $C(CH_3)_3$ | zähfl.Oel |
| 21 | $4-C=N-OCH_3$ 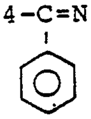 | - | N | CO | $C(CH_3)_3$ | :88-92°C |
| 22 | $4-C=N-OCH_3$ 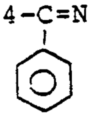 | - | CH | CO | $C(CH_3)_3$ | $n_D^{20}$:1,5542 |
| 23 | $4-CH=N-OCH_3$ | - | N | CO | 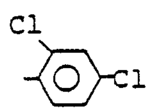 | zähflüssiges Oel |
| 24 | $4-\underset{CH_3}{C}=N-OCH_3$ | - | N | CO | 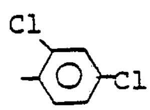 | zähflüssiges Oel |
| 25 | $4-CH=N-OCH_3$ | $2-OCH_3$ | N | CO | 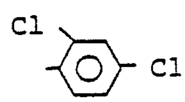 | zähflüssiges Oel |
| 26 | $4-C=N-OCH_3$ 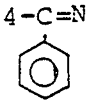 | - | N | CH(OH) | $C(CH_3)_3$ | glasartig |
| 27 | $4-\underset{CH_3}{C}=N-OCH_3$ | - | CH | CO | $C(CH_3)_3$ | $n_D^{20}$: 1,5421 |
| 28 | $4-CH=N-OCH_3$ | - | N | CH(OH) | 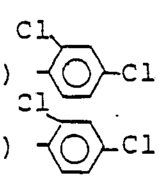 | 50-55 |
| 29 | $4-CH=N-OCH_3$ | $2-OCH_3$ | N | CH(OH) |  | 53-54 |
| 30 | $4-CH=N-OC_2H_5$ | - | N | CO | $C(CH_3)_3$ | zähflüssiges Oel |
| 31 | $4-CH=N-OC_2H_5$ | - | N | CH(OH) | $C(CH_3)_3$ | 93-96 |
| 32 | $4-CH=N-OC_2H_5$ | - | CH | CO | $C(CH_3)_3$ | zähflüssiges Oel |

In den nachfolgenden Beispielen werden die nachstehend angegebenen Verbindungen als Vergleichssubstanzen eingesetzt :

(A) = 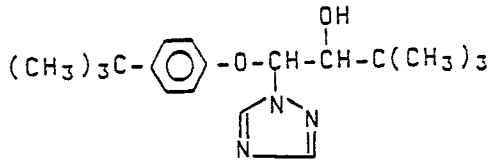 (DE-B-2324010)

(B) = 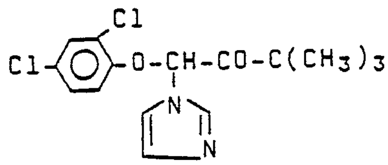 (DE-A-2325156)

(C) = 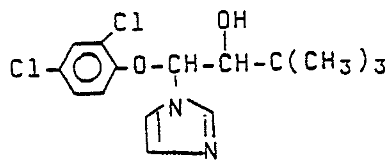 (DE-A-2333354)

(D) = 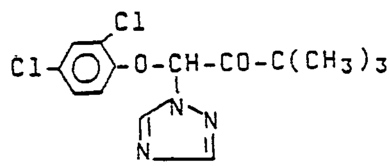 (DE-B-2201063)

(E) = 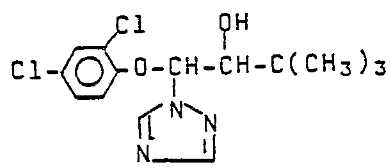 (DE-B-2324010)

(F) = 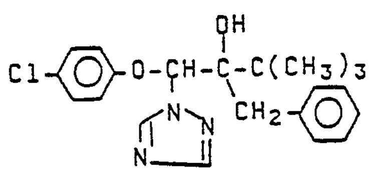 (DE-B-2324010)

Beispiel A

Erysiphe-Test (Gerste)/Saatgutbehandlung

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glasflasche.

Die Gerste sät man mit 3 × 12 Korn 2 cm tief in eine Standarderde. 7 Tage nach der Aussaat, wenn die jungen Pflanzen ihr erstes Blatt entfaltet haben, werden sie mit Sporen von Erysiphe graminis f. sp. hordei bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele : 2 und 11.

Beispiel B

Drechslera graminea-Test (Gerste)/Saatgutbehandlung (syn. Helminthosporium gramineum)

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das infizierte Saatgut 3 Minuten lang mit dem Beizmittel in einer verschlossenen Glasflasche.

Das Saatgut setzt man in gesiebter, feuchter Standarderde eingebettet, in verschlossenen Petrischalen im Kühlschrank 10 Tage lang einer Temperatur von 4 °C aus. Dabei wird die Keimung der Gerste und gegebenenfalls auch der Pilzsporen eingeleitet. Anschließend sät man die vorgekeimte Gerste mit 2 × 50 Korn 3 cm tief in eine Standarderde und kultiviert sie im Gewächshaus bei einer Temperatur von ca. 18 °C in Saatkästen, die täglich 15 Stunden dem Licht ausgesetzt werden.

Ca. 3 Wochen nach der Aussaat erfolgt die Auswertung der Pflanzen auf Symptome der Streifenkrankheit.

Eine deutliche Überlegenheit in der Wirksamkeit gegenüber dem Stand der Technik zeigen bei diesem Test z. B. die Verbindungen gemäß folgender Herstellungsbeispiele : 1.

## Beispiel C

Pyrenophora teres-Test (Gerste)/protektiv

Lösungsmittel : 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20 °C und 100 % relativer Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Eine deutliche Ueberlegenheit in der Wirksamkeit gegenüber dem Stand der Technik zeigen bei diesem Test z. B. die Verbindungen gemäß folgender Herstellungsbeispiele : 1, 4, 2 und 11.

## Beispiel D

Uromyces-Test (Buschbohne)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22 °C und 100 % relativer Luftfeuchtigkeit.

Die Pflanzen werden dann unter intensiver Belichtung für 9 Tage bei 20 bis 22 °C und einer relativen Luftfeuchtigkeit von 70 bis 80 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele : 4,2 und 11.

## Beispiel E

Venturia-Test (Apfel)/protektiv

Lösungsmittel : 4,7 Gewichtsteile Aceton
Emulgator : 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20 °C und 100 % relativer Luftfeuchtigkeit in einer Inkubationskabine.

Die Pflanzen werden dann im Gewächshaus bei 20 °C und einer relativen Luftfeuchtigkeit von ca. 70 % aufgestellt.

12 Tage nach der Inokulation erfolft die Auswertung.

Eine deutliche Überlegenheit in der Wirksamkeit gegenüber dem Stand der Technik zeigen in diesem Test z. B. die Verbindungen gemäß folgender Herstellungsbeispiele : 1, 4, 2 und 11.

**Patentansprüche**

1. Substituierte Azolyl-phenoxy-Derivate der allgemeinen Formel (I)

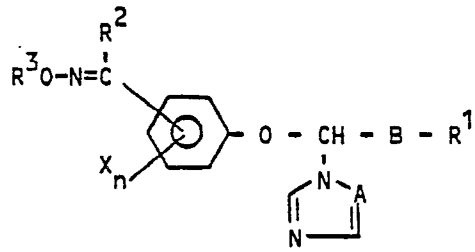

(I)

in welcher

A für ein Stickstoffatom oder die CH-Gruppe steht,

B für die Ketogruppe oder die CH(OH)-Gruppierung steht,

$R^1$ für Alkyl mit 1 bis 4 Kohlenstoffatomen und für Halogenalkyl mit 1 bis 4 Kohlenstoffatomen und 1 bis 3 Halogenatomen steht, sowie für gegebenenfalls durch Halogen, Alkyl mit 1 bis 4 Kohlenstoffatomen, Alkoxy und Alkylthio mit jeweils 1 bis 2 Kohlenstoffatomen und/oder Halogenalkyl mit 1 bis 2 Kohlenstoff- und 1 bis 5 gleichen oder verschiedenen Halogenatomen substituiertes Phenyl steht,

$R^2$ für Wasserstoff, für Alkyl mit 1 bis 4 Kohlenstoffatomen oder für gegebenenfalls durch die bei $R^1$ genannten Phenyl-Substituenten substituiertes Phenyl steht,

$R^3$ für Wasserstoff, für geradkettiges oder verzweigtes Alkyl mit 1 bis 4 Kohlenstoffatomen oder für Alkenyl und Alkinyl mit jeweils 2 bis 4 Kohlenstoffatomen steht,

X für Halogen, für Alkyl, Alkoxy und Alkylthio mit jeweils 1 bis 4 Kohlenstoffatomen oder für Halogenalkyl, Halogenalkoxy und Halogenalkylthio mit jeweils 1 bis 2 Kohlenstoffatomen und 1 bis 5 gleichen oder verschiedenen Halogenatomen steht, und

n für die Zahlen 0, 1 oder 2 steht,

sowie deren Säureadditions-Salze und Metallsalz-Komplexe.

2. Verbindungen der allgemeinen Formel (I) in Anspruch 1, in welcher

$R^1$ für tert.-Butyl, Chlor-tert.-butyl, Fluor-tert.-butyl, Dichlor-tert.-butyl, Difluor-tert.-butyl oder für gegebenenfalls einfach oder zweifach, gleich oder verschieden durch Fluor, Chlor und/oder Methyl substituiertes Phenyl steht ;

$R^2$ für Wasserstoff, Methyl, Ethyl, Isopropyl, oder für gegebenenfalls einfach oder zweifach, gleich oder verschieden durch Fluor, Chlor, Methyl, Methoxy und/oder Trifluormethyl substituiertes Phenyl steht ;

$R^3$ für Wasserstoff, Methyl, Ethyl, n-Propyl, n-Butyl, Allyl oder Propargyl steht ;

X für Fluor, Chlor, Brom, Jod, Methyl, Methoxy, Methylthio, Trifluormethyl, Trifluormethoxy oder Trifluormethylthio steht, und

A, B und n die in Anspruch 1 angegebenen Bedeutungen besitzen.

3. Verfahren zur Herstellung von substituierten Azolylphenoxy-Derivaten der allgemeinen Formel (Ia)

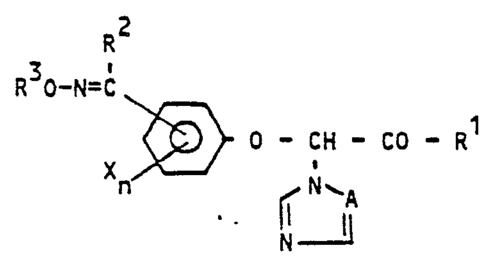

Ia

in welcher

A für ein Stickstoffatom oder die CH-Gruppe steht,

$R^1$ für Alkyl mit 1 bis 4 Kohlenstoffatomen und für Halogenalkyl mit 1 bis 4 Kohlenstoffatomen und 1 bis 3 Halogenatomen steht, sowie für gegebenenfalls durch Halogen, Alkyl mit 1 bis 4 Kohlenstoffatomen, Alkoxy und Alkylthio mit jeweils 1 bis 2 Kohlenstoffatomen und/oder Halogenalkyl mit 1 bis 2 Kohlenstoff- und 1 bis 5 gleichen oder verschiedenen Halogenatomen substituiertes Phenyl steht,

$R^2$ für Wasserstoff, für Alkyl mit 1 bis 4 Kohlenstoffatomen oder für gegebenenfalls durch die bei $R^1$ genannten Phenyl-Substituenten substituiertes Phenyl steht,

$R^3$ für Wasserstoff, für geradkettiges oder verzweigtes Alkyl mit 1 bis 4 Kohlenstoffatomen oder für Alkenyl und Alkinyl mit jeweils 2 bis 4 Kohlenstoffatomen steht,

X für Halogen, für Alkyl, Alkoxy und Alkylthio mit jeweils 1 bis 4 Kohlenstoffatomen oder für Halogenalkyl, Halogenalkoxy und Halogenalkylthio mit jeweils 1 bis 2 Kohlenstoffatomen und 1 bis 5 gleichen oder verschiedenen Halogenatomen steht, und

n für die Zahlen 0, 1 oder 2 steht,

dadurch gekennzeichnet, daß man

a) Halogenetherketone der Formel (II)

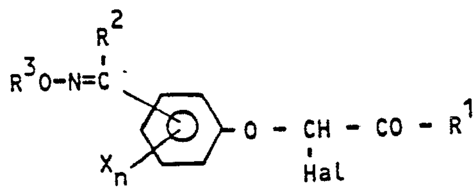

(II)

in welcher

$R^1$, $R^2$, $R^3$, X und n die oben angegebene Bedeutung haben und

Hal für Halogen steht,

mit 1,2,4-Triazol oder Imidazol, gegebenenfalls in Form von Alkalimetallsalzen, gegebenenfalls in Gegenwart eines Säurebindemittels und gegebenenfalls in Gegenwart eines Verdünnungsmittels umsetzt, oder

b) Azolylhalogenketone der Formel (III)

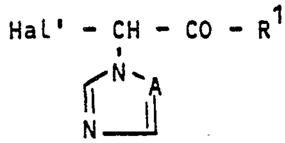

(III)

in welcher

A und $R^1$ die oben angegebene Bedeutung haben und

Hal' für Halogen steht,

mit Phenolen der Formel (IV)

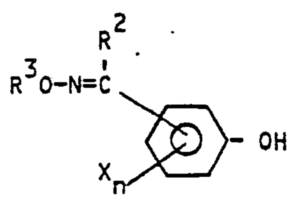

(IV)

in welcher $R^2$, $R^3$, X und n die oben angegebene Bedeutung haben, in Gegenwart eines Säurebindemittels und in Gegenwart eines Verdünnungsmittels umsetzt, und an die erhaltenen Verbindungen der Formel (Ia) gegebenenfalls noch anschließend eine Säure oder ein Metallsalz addiert.

4. Verfahren zur Herstellung von substituierten Azolylphenoxy-Derivaten der allgemeinen Formel (Ib)

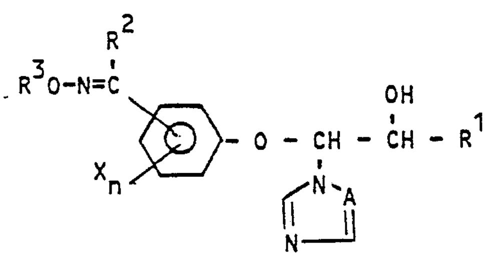

Ib

in welcher A, $R^1$, $R^2$, $R^3$, X und n die in Anspruch 3 angegebene Bedeutung besitzen, dadurch gekennzeichnet, daß man die Verbindungen der Formel (Ia) in Anspruch 3 nach bekannten Methoden in üblicher Weise reduziert, und an die erhaltenen Verbindungen der Formel (Ib) gegebenenfalls noch anschließend eine Säure oder ein Metallsalz addiert.

5. Fungizide Mittel, gekennzeichnet durch einen Gehalt an mindestens einem substituierten Azolyl-phenoxy-Derivat der Formeln (I), (Ia) und (Ib) gemäß den Ansprüchen 1, 3 und 4.

6. Verwendung von substituierten Azolyl-phenoxy-Derivaten der Formeln (I), (Ia) und (Ib) gemäß den Ansprüchen 1, 3 und 4 zur Bekämpfung von phytopathogenen Pilzen.

**Claims**

1. Substituted azolyl-phenoxy derivatives of the general formula (I)

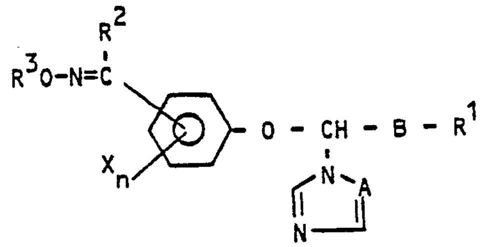

(I)

in which

A represents a nitrogen atom or the CH group,

B represents the keto group or the CH(OH) grouping,

$R^1$ represents alkyl with 1 to 4 carbon atoms and halogenoalkyl with 1 to 4 carbon atoms and 1 to 3 halogen atoms, and phenyl which is optionally substituted by halogen, alkyl with 1 to 4 carbon atoms, alkoxy und alkylthio each with 1 to 2 carbon atoms and/or halogenoalkyl with 1 to 2 carbon and 1 to 5 identical or different halogen atoms,

$R^2$ represents hydrogen, alkyl with 1 to 4 carbon atoms or phenyl which is optionally substituted by the phenyl substituents mentioned under $R^1$,

$R^3$ represents hydrogen, straight-chain or branched alkyl with 1 to 4 carbon atoms or alkenyl and alkinyl each with 2 to 4 carbon atoms,

X represents halogen, alkyl, alkoxy and alkylthio each with 1 to 4 carbon atoms or halogenoalkyl, halogenoalkoxy and halogenoalkylthio each with 1 to 2 carbon atoms and 1 to 5 identical or different halogen atoms, and

n represents the numbers 0, 1 or 2,

and acid addition salts and metal salt complexes thereof.

2. Compounds of the general formula (I) in Claim 1, in which

$R^1$ represents tert.-butyl, chloro-tert.-butyl, fluoro-tert.-butyl, dichloro-tert.-butyl, difluoro-tert.-butyl or phenyl which is optionally mono- or disubstituted by identical or different substituents selected from fluorine, chlorine and/or methyl ;

$R^2$ represents hydrogen, methyl, ethyl, isopropyl or phenyl which is optionally mono- or disubstituted by identical or different substituents selected from fluorine, chlorine, methyl, methoxy and/or trifluoromethyl ;

$R^3$ represents hydrogen, methyl, ethyl, n-propyl, n-butyl, allyl or propargyl ;

X represents fluorine, chlorine, bromine, iodine, methyl, methoxy, methylthio, trifluoromethyl, trifluoromethoxy or trifluoromethylthio, and

A, B and n have the meanings given in Claim 1.

3. Process for the preparation of substituted azolyl-phenoxy derivatives of the general formula (Ia)

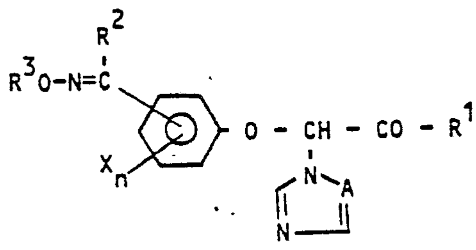

Ia

in which

A represents a nitrogen atom or the CH group,

$R^1$ represents alkyl with 1 to 4 carbon atoms and halogenoalkyl with 1 to 4 carbon atoms and 1 to 3 halogen atoms, and phenyl which is optionally substituted by halogen, alkyl with 1 to 4 carbon atoms, alkoxy and alkylthio each with 1 to 2 carbon atoms and/or halogenoalkyl with 1 to 2 carbon and 1 to 5 identical or different halogen atoms,

$R^2$ represents hydrogen, alkyl with 1 to 4 carbon atoms or phenyl which is optionally substituted by the phenyl substituents mentioned under $R^1$,

$R^3$ represents hydrogen, straight-chain or branched alkyl with 1 to 4 carbon atoms or alkenyl and alkinyl each with 2 to 4 carbon atoms,

X represents halogen, alkyl, alkoxy and alkylthio each with 1 to 4 carbon atoms or halogenoalkyl, halogenoalkoxy and halogenoalkylthio each with 1 to 2 carbon atoms and 1 to 5 identical or different halogen atoms, and

n represents the numbers 0, 1 or 2,

characterised in that

a) halogenoether ketones of the formula (II)

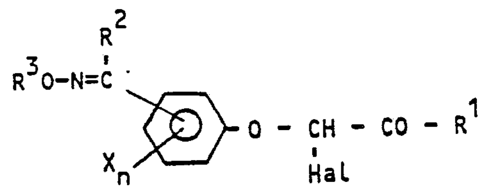

(II)

in which

$R^1$, $R^2$, $R^3$, X and n have the abovementioned meaning and

Hal represents halogen,

are reacted with 1,2,4-triazole or imidazole, if appropriate in the form of alkali metal salts, if appropriate in the presence of an acid-binding agent and if appropriate in the presence of a diluent, or

b) azolylhalogenoketones of the formula (III)

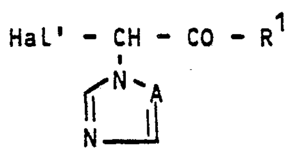

(III)

in which

A and $R^1$ have the abovementioned meaning and

Hal' represents halogen,

are reacted with phenols of the formula (IV)

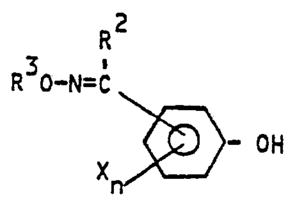

(IV)

in which $R^2$, $R^3$, X and n have the abovementioned meaning, in the presence of an acid-binding agent and in the presence of a diluent, and, if desired, an acid or a metal salt is then also added on to the compounds obtained of the formula (Ia).

4. Process for the preparation of substituted azolyl-phenoxy derivatives of the general formula (Ib)

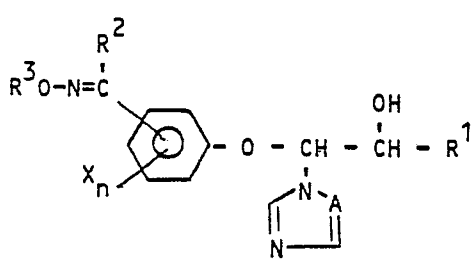

Ib

in which A, $R^1$, $R^2$, $R^3$, X and n have the meaning given in Claim 3, characterised in that the compounds of

the formula (Ia) in Claim 3 are reduced in the customary manner by known methods and, if desired, an acid or a metal salt is then also added on to the compounds obtained of the formula (Ib).

5. Fungicidal agents, characterised in that they contain at least one substituted azolyl-phenoxy derivative of the formulae (I), (Ia) and (Ib) according to Claims 1, 3 and 4.

6. Use of substituted azolyl-phenoxy derivatives of the formulae (I), (Ia) and (Ib) according to Claims 1, 3 and 4 for combating phytopathogenic fungi.

**Revendications**

1. Dérivés azolyl-phénoxyliques substitués de formule générale I

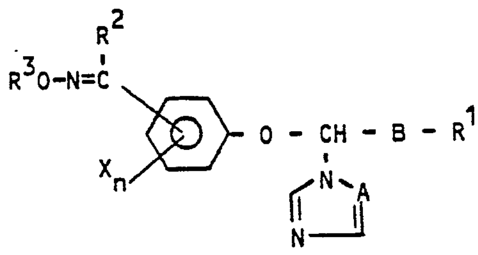

(I)

dans laquelle

A représente un atome d'azote ou le groupe CH,

B représente le groupe céto ou le groupement CH(OH),

$R^1$ représente un groupe alkyle en $C_1$-$C_4$ ou halogénoalkyle contenant 1 à 4 atomes de carbone et 1 à 3 atomes d'halogènes, ou phényle éventuellement substitué par des halogènes, des groupes alkyle en $C_1$-$C_4$, alcoxy et alkylthio contenant chacun 1 à 2 atomes de carbone et/ou halogénoalkyle contenant 1 à 2 atomes de carbone et 1 à 5 atomes d'halogènes identiques ou différents,

$R^2$ représente l'hydrogène, un groupe alkyle en $C_1$-$C_4$ ou un groupe phényle portant éventuellement les substituants mentionnés pour ce groupe en référence à $R^1$,

$R^3$ représente l'hydrogène, un groupe alkyle à chaîne droite ou ramifiée en $C_1$-$C_4$ ou alcényle ou alcynyle contenant chacun 2 à 4 atomes de carbone,

X représente un halogène, un groupe alkyle, alcoxy ou alkylthio contenant chacun 1 à 4 atomes de carbone ou halogénoalkyle, halogénoalcoxy ou halogénoalkylthio contenant chacun 1 à 2 atomes de carbone et 1 à 5 atomes d'halogènes identiques ou différents, et

n est égal à 0, 1 ou 2,

ainsi que leurs sels formés par addition avec des acides et complexes de sels métalliques.

2. Composés de formule générale I selon la revendication 1, dans lesquels :

$R^1$ représente un groupe tert-butyle, chloro-tert-butyle, fluoro-tert-butyle, dichloro-tert-butyle, difluoro-tert-butyle ou phényle portant éventuellement 1 ou 2 substituants identiques ou différents choisis parmi le fluor, le chlore et/ou les groupes méthyle ;

$R^2$ représente l'hydrogène, un groupe méthyle, éthyle, isopropyle ou phényle portant éventuellement 1 ou 2 substituants identiques ou différents choisis parmi le fluor, le chlore, les groupes méthyle, méthoxy et/ou trifluorométhyle ;

$R^3$ représente l'hydrogène, un groupe méthyle, éthyle, n-propyle, n-butyle, allyle ou propargyle ;

X représente le fluor, le chlore, le brome, l'iode, un groupe méthyle, méthoxy, méthylthio, trifluorométhyle, trifluorométhoxy ou trifluorométhylthio ; et

A, B et n ont les significations indiquées dans la revendication 1.

3. Procédé de préparation de dérivés azolyl-phénoxyliques substitués de formule générale Ia

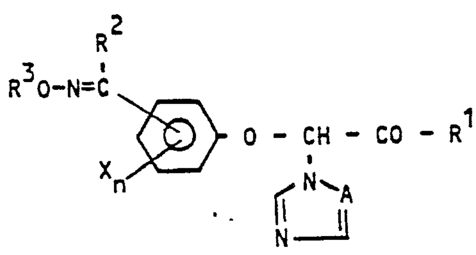

Ia

dans laquelle

A représente un atome d'azote ou le groupe CH,

$R^1$ représente un groupe alkyle en $C_1$-$C_4$ ou halogénoalkyle contenant 1 à 4 atomes de carbone et 1 à 3 atomes d'halogènes, ou encore phényle éventuellement substitué par des halogènes, des groupes

alkyle en $C_1$-$C_4$, alcoxy et alkylthio contenant chacun 1 à 2 atomes de carbone et/ou halogénoalkyle contenant 1 à 2 atomes de carbone et 1 à 5 atomes d'halogènes identiques ou différents,

$R^2$ représente l'hydrogène, un groupe alkyle en $C_1$-$C_4$ ou phényle portant éventuellement les substituants mentionnés pour le groupe phényle en référence à $R^1$,

$R^3$ représente l'hydrogène, un groupe alkyle à chaîne droite ou ramifiée en $C_1$-$C_4$ ou alcényle ou alcynyle contenant chacun 2 à 4 atomes de carbone,

X représente un halogène, un groupe alkyle, alcoxy ou alkylthio contenant chacun 1 à 4 atomes de carbone ou halogénoalkyle, halogénoalcoxy et halogénoalkylthio contenant chacun 1 à 2 atomes de carbone et 1 à 5 atomes d'halogènes identiques ou différents, et

n est égal à 0, 1 ou 2,

ce procédé se caractérisant en ce que :

a) on fait réagir des halogéno-éther-cétones de formule II

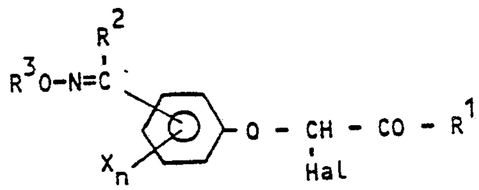

(II)

dans laquelle

$R^1$, $R^2$, $R^3$, X et n ont les significations indiquées ci-dessus, et

Hal représente un halogène,

avec le 1,2,4-triazole ou l'imidazole, éventuellement à l'état de sels de métaux alcalins, éventuellement en présence d'un agent fixant les acides et éventuellement en présence d'un diluant, ou bien

b) on fait réagir des azolylhalogénocétones de formule III

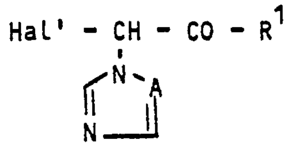

(III)

dans laquelle

A et $R^1$ ont les significations indiquées ci-dessus, et

Hal' représente un halogène,

avec des phénols de formule IV

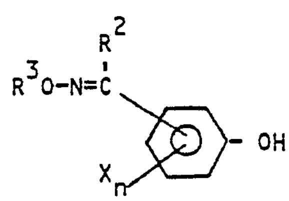

(IV)

dans laquelle $R^2$, $R^3$, X et n ont les significations indiquées ci-dessus, en présence d'un agent fixant les acides et en présence d'un diluant et, sur les composés de formule Ia ainsi obtenus, on fixe encore, le cas échéant, par addition un acide ou un sel métallique.

4. Procédé de préparation de dérivés azolyl-phénoxyliques substitués de formula générale Ib

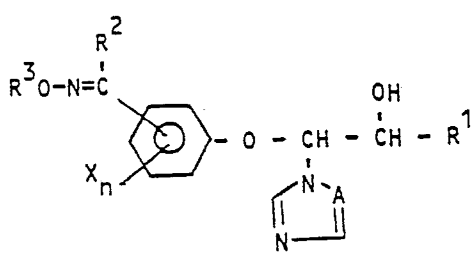

Ib

dans laquelle A, $R^1$, $R^2$, $R^3$, X et n ont les significations indiquées dans la revendication 3, caractérisé en ce que l'on réduit de la manière habituelle, par des modes opératoires connus, les composés de formule

la de la revendication 3 et, sur les composés de formule Ib ainsi obtenus, on fixe encore par addition, le cas échéant, un acide ou un sel métallique.

5. Produits fongicides, caractérisés en ce qu'ils contiennent au moins un dérivé azolyl-phénoxylique substitué de formules I, Ia ou Ib selon les revendications 1, 3 et 4.

6. Utilisation des dérivés azolyl-phénoxyliques substitués de formules I, Ia et Ib selon les revendications 1, 3 et 4 dans la lutte contre les mycètes phytopathogènes.